# EUROPEAN PATENT APPLICATION

(11) **EP 2 657 866 A1**
(43) Date of publication of application: **30.10.2013**
(21) Application number: 12165355.4
(22) Date of filing: 24.04.2012
(51) Int. Cl.: G06F 19/00

(54) **Creating a radiology report**

(71) Applicant: Koninklijke Philips N.V., 5656 AE Eindhoven (NL)
(72) Inventor: Serlie, Iwo Willem Oscar, 5600 AE Eindhoven (NL); Aleksovski, Zarko, 5600 AE Eindhoven (NL)
(74) Representative: Damen, Daniel Martijn

(57) **Abstract**

A system for creating a report comprises a report unit (1) for enabling a user to create a report (21). A marking storing unit (2) is arranged for storing a collection of markings, wherein a marking (301) represents an annotation (302) and a set of viewing parameters (306) in respect of an image dataset. A marking selection unit (3) is arranged for enabling a user to select a marking (301) of the collection of markings for display or insertion. An image display unit (4) is arranged for, in response to the user selecting the marking (301) for display, displaying the image dataset in dependence on the viewing parameters (306). An inserting unit (5) is arranged for, in response to the user selecting the marking (301) for insertion, inserting information into the report in dependence on the marking (301).

## Description

### FIELD OF THE INVENTION

The invention relates to clinical decision support. The invention further relates to creating a report, in particular a radiology report. The system further relates to displaying an image dataset.

### BACKGROUND OF THE INVENTION

An important purpose of a radiology report is to answer a clinical question asked by a referring physician by means of a radiology request. The report can be an effective way to convey such information to the reader of the report. Words are considered a suitable way to transfer the expertise of the radiologist, applied to a specific radiological examination, to the referring physician.

In clinical practice, the radiologist or a resident may prepare the reporting process by creating image markings. The radiologist reviews the markings and dictates the report. The image markings are not necessarily created by the same radiologist who dictates the report. These markings may also be created by a resident, for example. Alternatively, the radiologist may create the markings during the reporting.

### SUMMARY OF THE INVENTION

It would be advantageous to have an improved system for displaying an image dataset. To better address this concern, a first aspect of the invention provides a system comprising
a marking storing unit for storing a collection of markings, wherein a marking represents an annotation and a set of viewing parameters in respect of an image dataset;
a marking selection unit for selecting a marking of the collection of markings for display; and
an image display unit for, in response to the marking being selected for display, displaying the image dataset in dependence on the viewing parameters of the marking selected for display.

The system allows an efficient handling of markings in radiology reporting. The user does not need to manually look up the image markings by manipulating the viewing parameters. Instead, the image dataset is displayed in dependence on the relevant viewing parameters by simply selecting a marking from the collection of markings. Currently, the radiologist is expected to create annotations during reporting and, when the radiologist is able to make annotations before reporting, he has to navigate through the images to find back any markings during reporting. By the collection of markings, these annotations and associated images are easily accessible.

The marking selection unit may be further arranged for selecting the marking of the collection of markings for insertion. Moreover, the system may comprise a report unit for creating a report, and an inserting unit for, in response to the marking being selected for insertion, inserting information into the report in dependence on the marking. When the information about the marking is needed in the report, the user can insert the information of a marking into the report by selecting the marking for insertion. This integration of markings with the reporting process makes drafting the report more efficient. For example, preparation of the report may be done by a resident by creating markings with suitable annotations. However, because these annotations are stored in the collection of markers, the radiologist can then make the decision whether or not to include the information into the report. This makes it possible to separate the process of creating markings or attention points and the report creation. The collaboration between persons that read and report the images may be improved in this way.

The system may comprise a marking creation unit for creating a marking while the image dataset is displayed, and providing the marking to the marking storage unit for storage. This allows a user to add markings more easily.

The marking creation unit may comprise an annotation creation unit for enabling the user to indicate the annotation and a parameter unit for determining the set of viewing parameters of the marking based on a displayed view of the image dataset. This way, the set of viewing parameters is determined automatically, based on the displayed view. For example, the viewing parameters of the displayed view may be copied into the marking. Moreover, the user is allowed to create an annotated marking, so that sufficient information is made available in the marking to enable the radiologist to perform the reporting efficiently.

The marking creation unit may comprise a template for enabling the user to provide information in respect of a marking in a structured form. For example, a plurality of templates may be available, enabling the user to generate different kinds of markings according to the circumstances. Moreover, the template makes the markings more uniform and user independent.

The system may support a first operating mode in which the marking creation unit is enabled and the inserting unit is disabled. The system may also support a second operating mode in which the inserting unit is enabled. This allows to distinguish between the preparatory phase of marking creation, which may involve a relatively quick scan of the image data, and the reporting phase, in which the final reporting is generated, after examining the marked image portions and annotations in more detail.

The operating mode may be determined in dependence on an authorization privilege of the user. This allows for example the annotations to be generated by a less senior staff member compared to the staff member who is given an authorization privilege to create elements of the report.

The system may comprise a marking interface unit for displaying at least part of the collection of markings. The marking selection unit may be arranged for enabling the user to perform the selection of a marking via the marking interface unit. For example, a window may be provided that allows the user to scroll through the collection of markings and click on a marking or touch a marking to select it. This allows the user to operate the system efficiently. Moreover, this markings window may be displayed concurrently with a view of an appropriate image dataset. Moreover, a window showing the report and/or allowing a user to scroll through a report and insert information into the report, may be displayed concurrently with the markings window and the view of the image dataset. This provides an efficient overview of information needed for report creation.

The marking interface unit may be arranged for displaying markings in respect of a plurality of different image datasets, and for displaying an indication of which marking belongs to which image dataset. This enhances the system in case the markings relate to different image datasets. For example, this allows the collection of markings to provide markings of different image datasets of the same patient. In such a way, progression of disease can be monitored more easily and in a more or less ad hoc way, before creation of the report, using the collection of markings.

The annotation of a marking may comprise a graphical object or a measurement defined in respect of the image dataset. The display unit may be arranged for, in response to the marking being selected for display, displaying the graphical object or measurement in association with the image dataset. This allows such annotations as measurements or pointers to objects in the image dataset, to be organized in the collection of markings. A measurement may involve a graphical object that is aligned with a specific structure in the image dataset. Examples of measurements include a length measurement and a volume measurement. These measurements may be performed automatically, semiautomatically, or manually. The inserting unit may be arranged for inserting an image of the graphical object or measurement into the report.

The annotation of a marking may comprise a text fragment. The inserting unit may be arranged for pasting the text fragment into the report in response to the marking being selected for insertion. This may increase efficiency and reduce errors.

The annotation of a marking may comprise a measurement defined in respect of the image dataset. The inserting unit may be arranged for pasting information relating to the measurement into the report in response to the user selecting the marking for insertion. This makes the reporting more efficient and may reduce errors in copying information relating to the measurement. For example, the inserting unit may be arranged for inserting a quantitative result of the measurement into the report.

The inserting unit may be arranged for inserting said at least part of the marking into the report as a structured data element. This helps to make the report more structured. Moreover, such structured data elements may be used later on for automatic processing of the report by other clinical support systems.

The inserting unit may be arranged for inserting a plurality of markings into the report as at least part of a table. This is convenient, for example when the plurality of markings are related to each other, for example it is a standard set of measurements that is performed on an image. Such a table may also be suitable when the markings span multiple image datasets, and corresponding markings exist for two or more image datasets. In such a case, corresponding markings may be arranged in columns, on column for each dataset. Other arrangements are also possible.

The inserting unit may be arranged for inserting the information at a position of the report that depends on the marking. This improves efficiency of report creation. Moreover, it may improve reproducibility of reports. For example, a tumor finding with certain properties, as recorded in the annotation of a marking, may be inserted automatically at the appropriate position of the report, for example the findings section. In some cases, the information available in the marking may be distributed among the report. Some information may be inserted in one section, and some information of the same marking may be inserted in a different section. For example, information may be automatically distributed over the findings section and the impressions section.

In another aspect, the invention provides a workstation comprising a system set forth.

In another aspect, the invention provides a method of creating a radiology report, comprising
storing a collection of markings, wherein a marking represents an annotation and a set of viewing parameters in respect of an image dataset;
selecting a marking of the collection of markings for display;
in response to the marking being selected for display, displaying the image dataset in dependence on the viewing parameters of the marking selected for display.

In another aspect, the invention provides a computer program product comprising instructions for causing a processor system to perform the method set forth.

In another aspect, the invention provides a computer program product representing an implementation of a system set forth.

It will be appreciated by those skilled in the art that two or more of the above-mentioned embodiments, implementations, and/or aspects of the invention may be combined in any way deemed useful.

Modifications and variations of the image acquisition apparatus, the workstation, the method, and/or the computer program product, which correspond to the described modifications and variations of the system, can be carried out by a person skilled in the art on the basis of the present description.

### BRIEF DESCRIPTION OF THE DRAWINGS

These and other aspects of the invention are apparent from and will be elucidated hereinafter with reference to the drawings. The drawings are diagrammatic and may not be drawn to scale.
Fig. 1 is a block diagram of a system for creating a report.
Fig. 2 is a flowchart of a method of creating a report.
Fig. 3 is a diagram showing elements of a marking.
Fig. 4 is a screenshot of a system for creating a report.

### DETAILED DESCRIPTION OF EMBODIMENTS

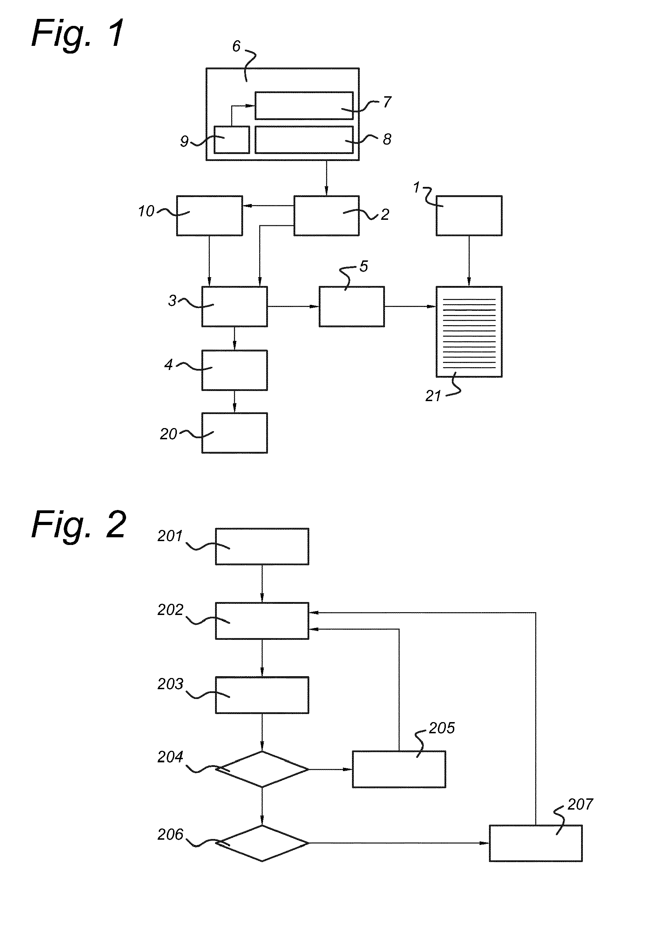

Fig. 1 shows a block diagram of a system for creating a report. The system may be implemented as a suitably programmed computer system. Alternatively, the system may be implemented by means of dedicated electronic circuitry. The system may comprise one or more user interface devices, such as a keyboard, pointing device, microphone, and/or touch screen. Moreover, the system may comprise a display for displaying image data, annotations, report fragments, control buttons, and more. The system may further comprise a communications port for providing electronic communication with other devices, for example via a network. The communications port may be used for receiving and transmitting for example image datasets, annotations, markings, and reports. The system may comprise storage means for storing for example image datasets, annotations, markings, and reports.

It is noted that the units of the system relating to the actual creation of the report may be omitted. This way, a system is obtained that can be used to display the image dataset using the markings.

The system may comprise a report unit 1 arranged for enabling a user to create a report 21. For example, the report unit enables the user to provide text input. Such text input may be provided by means of a keyboard and/or by means of dictation. In the latter case, voice recognition may be employed to translate the voice input into text before inclusion into the report. The report may further comprise graphical elements, such as images or drawings. The text may be formatted, using different fonts, or tables, for example, in a way known in the art per se. Alternatively, the creation of the report may be performed partially or completely automatically.

The system may further comprise a marking storing unit 2 arranged for storing a collection of markings. These markings 301 may be stored locally in a memory. Additionally or alternatively, the markings may be transferred via the communications port to a server system to be stored permanently.

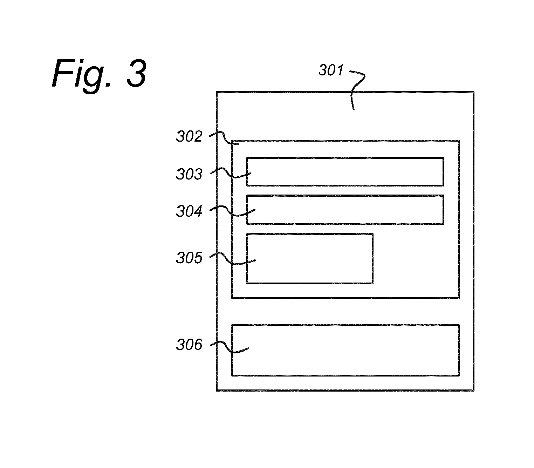

Fig. 3 shows a diagram comprising possible elements of such a marking 301. The marking 301 comprises a representation of an annotation 302 and a representation of a set of viewing parameters 306. These viewing parameters 306 may be defined in respect of a particular image dataset. The viewing parameters 306 may comprise a reference to the relevant image dataset. Alternatively, this reference may be defined elsewhere. The viewing parameters may define aspects of a visualization of the image dataset. Such viewing parameters may include, but are not limited to: window width/window level, orientation, rotation, translation, zoom level, slice number, position and/or orientation of a cutting plane. The viewing parameters may also define the display mode, such as maximum intensity projection, volume rendering, or slice view in case the image dataset comprises a volume dataset. However, the image dataset may also comprise a two-dimensional image.

The annotation 302 may comprise a text portion 305. Such a text portion may comprise a free text provided by a user at the time of creating the marking. The text portion may also comprise a label or a standard text associated with a particular kind of marking. This may be defined by a template, as will be described hereinafter.

The annotation 302 may also comprise an indication of a graphical object 303. This graphical object 303 could be an arrow, a circle, or any graphical object. The indication of the graphical object 303 may comprise coordinates and/or orientation parameters of the graphical object 303, defined in respect of the image dataset. This way, the graphical object can be used to indicate a particular structure in the image dataset. The graphical object may be positioned manually by a user at the time of creating the marking 301, or in an automated or computer-aided manner. In the latter case, for example automatic feature extraction techniques may be used to detect a particular type of object in the image dataset, and position the graphical object to point out the object.

The annotation 302 may also comprise an indication of a measurement 304. Such a measurement may also involve a graphical object. A measurement 304 may comprise an outcome, in particular a quantification such as a numeric value. The way to perform a measurement may be predetermined. One or more user-operable measurement tools may be provided to help the user to perform a measurement. Such measurement tools may be operatively connected to the marking storing unit 2 to store measurement parameters in the measurement 304 portion of the marking 301. The measurement may be related to a dimension, such as a distance or a volume measurement, defined in respect of the image dataset. The measurement may also be related to gray values or colors in the image dataset. The measurement may be local, in which case the measurement is related to a portion of the image. Such a portion may be indicated by means of a graphical object. Consequently, the measurement 304 may implicitly or explicitly define parameters of a graphical object defined in respect of the image dataset. In view of the present disclosure, the skilled person is able to implement different kinds of measurements, and store the relevant information relating to the measurements in a marking 301.

The system may comprise a marking selection unit 3 arranged for enabling a user to select a marking 301 of the collection of markings for display or insertion. For example, the user may select the marking 301 from a displayed list of markings. Alternatively, the markings may be assigned reference identifiers, and the user may select a marking by means of its reference identifier. Moreover, the user may choose between display and insertion by means of different buttons on a graphical user interface, for example. Alternatively, the selection of a marking may be performed automatically. For example, the markings may be selected for display in a sequential order. The user may be enabled to indicate whether or not a marking that is selected for display should be also selected for insertion.

The marking selection unit 3 may be operatively connected to an image display unit 4. The image display unit 4 may be arranged for, in response to the user selecting the marking 301 for display, displaying the image dataset in dependence on the viewing parameters 306. For example, the display unit 3 is arranged for retrieving the viewing parameters 306 from the selected marking 301. These viewing parameters 306 may be used to reproduce the view of the image dataset, with the same viewing parameters as used when creating the marking, for example the same window width/window level setting and/or the same zoom, translation, and/or rotation values. Moreover, the viewing parameters 306 may comprise a reference to the relevant image dataset. In the latter case, the image display unit 4 may be arranged for retrieving the referenced image dataset, applying the viewing parameters to the referenced image dataset, and displaying the referenced image dataset based on the viewing parameters. Moreover, the image display unit 4 may be arranged for checking if a representation of a graphical object 303 is present in the marking 301, and showing the graphical object 303 in its proper relation to the displayed image dataset. Moreover, the image display unit 4 may be arranged for checking if a measurement 304 is present in the marking 301, and displaying an associated graphical object in its proper relation to the displayed image dataset, according to the parameters and the type of the measurement 304.

The marking selection unit 3 may be operatively connected to an inserting unit 5. The inserting unit 5 may be arranged for, in response to the user selecting the marking 301 for insertion, inserting information into the report in dependence on the marking 301. This information may comprise the reference to the relevant image dataset, and any subset of the marking or the complete information comprised in the marking 301. Moreover, the inserting unit 5 may be arranged for generating information elements that are suitable for inclusion in the report 21, based on the marking 301. For example, the viewing parameters 306 may be used to generate a snapshot of the image dataset, and the snapshot may be included into the report. Moreover, the inserting unit 5 may comprise a natural language generator for generating a natural language portion based on the information in the marking 301. For example, if the marking comprises a particular kind of measurement, a written description of the kind of measurement and/or the outcome of the measurement may be generated.

The inserting unit 5 may be arranged for inserting the information at a cursor position indicated by the user. The inserting unit 5 may also be arranged for inserting the information at a position within the report that depends on the kind of information in the marking and on the image dataset to which the marking relates.

The marking selection unit 3 may be arranged for enabling the user to select a plurality of markings for inclusion into the report 21. The marking selection unit may be arranged for including such a selected group of markings into the report as a group.

In case a plurality of markings are inserted into the report, and some of the markings are related to different image datasets, the markings may be arranged in the report by the inserting unit 5 based on the image dataset to which they refer. If the image datasets are associated with a different date/time, the inserted markings may be sorted according to such date/time.

The system may comprise a marking creation unit 6 arranged for enabling a user to create a marking 301 in respect of an image dataset while that image dataset is displayed. The marking creation unit may be arranged for providing the marking 301 to the marking storage unit 2 for storage.

The marking creation unit 6 may comprise an annotation creation unit 7 arranged for enabling the user to indicate the annotation 302. Moreover, the marking creation unit 6 may comprise a parameter unit 8 arranged for determining the set of viewing parameters 306 of the marking based on a displayed view of the image dataset.

The marking creation unit 6 may comprise a template 9 for enabling the user to provide information in respect of a marking in a structured form. Such a template may prescribe what information should be provided by the user, what (if any) information should be obtained from the image dataset based on the information provided by the user, for example including a specification of any computations to be performed. The template may further prescribe which information should be stored into the marking 301 (and which information may be discarded after use).

The system may be configured to support a first operating mode in which the marking creation unit 6 is enabled and the inserting unit 5 is disabled. The system may further be configured to support a second operating mode in which the inserting unit 5 is enabled. In the second operating mode, the marking creation unit 6 may be enabled or disabled, according to need. Normally, it may be allowed to create new markings also during the creating of the report, for example if an issue has been overlooked. Consequently, in some applications, the marking creation unit may be enabled in the second operating mode. The first operating mode is useful for the preparatory phase, in which the user scans the available image dataset(s), and creates the markings for any clinically relevant image features. In this case, it may be useful to disable report creation in general, and the inserting unit 5 in particular, to avoid mistakes, because the user performing the preparatory work may not be authorized to perform actual reporting. Other parts of the system may be disabled in the first operating mode also, such as the report unit 1.

The system may be arranged for determining the operating mode in dependence on an authorization privilege of the user. To this end, the system may comprise an operating mode determining unit, which is not shown in the drawing. The operating mode determining unit may be operatively coupled to a user identification system that is usually external to the system described herein. The user identification system may be operable to identify the user of the system and supply information about the authorization privileges of the user. For example, some users are associated with a category "radiologist", whereas others are not. The system may be arranged for applying the second operating mode if the user is of category "radiologist", and applying the first operating mode if the user is not of category "radiologist".

The system may comprise a marking interface unit 10 arranged for displaying at least part of the collection of markings. For example, the marking unit allows the user to scroll through the collection of markings using a graphical user interface. The marking selection unit 3 may be arranged for enabling the user to perform the selection of a marking via the marking interface unit 10.

The marking interface unit 10 may be arranged for displaying the collection of markers in some different kinds of ways. Moreover, the marking storing unit 2 may be arranged for storing the collection of markings in different ways, compatible with the intended display thereof. For example, the collection of markings 2 may be stored as a rich text document, including the information of the marking 301 as metadata associated with a piece of text of the text document. Alternatively, the collection of markings 2 may be provided as an XML document, and the marking interface unit 10 comprises an XML style sheet that generates a visualization of the collection of markings based on the XML document.

The marking interface unit 10 may be arranged for displaying markings in respect of a plurality of different image datasets, and for displaying an indication of which marking belongs to which image dataset. For example, a marking may be displayed with an indication of the image dataset. In another example, markings may be arranged in columns or rows, one row or column for each dataset.

The annotation 302 may comprise a representation of a graphical object 303 or a representation of a measurement 304. Such graphical object or measurement may be defined in respect of the image dataset. The display unit 4 may be arranged for displaying the graphical object 303 or measurement 304 in association with the image dataset. For example, the display unit 4 may be arranged for displaying the image dataset based on the viewing parameters 306 of the marking 301 together with the graphical object 303 or a graphical indication of the measurement 304. This displaying may be performed in response to the user selecting the marking 301 for display,

As described above, the annotation 302 may comprise a text fragment 305. The inserting unit 5 may be arranged for pasting such a text fragment 305 into the report 21 1 in response to the user selecting the marking 301 for insertion.

As described above, the annotation 302 may comprise a representation of a measurement 304 defined in respect of the image dataset. The inserting unit 5 may be arranged for pasting information relating to the measurement 304 into the report 21 in response to the user selecting the marking for insertion. This information may include a description of the measurement, the location of the measurement, the measured value(s), and/or a snapshot of the image dataset based on the viewing parameters and with an indication of the measured structure(s) or image portion(s).

The inserting unit 5 may be arranged for inserting said at least part of the marking 301 into the report as a structured data element. For example, if the collection of markings comprises the individual markings as structured data elements, these elements could be copied into the report as a structured data element. The viewing parameters could be omitted in the report, in particular if the audience of the report does not have the ability to reproduce the image visualization based on the image parameters.

The inserting unit 5 may be arranged for inserting a plurality of markings into the report as at least part of a table. A plurality of markings may be related to each other, in such a way that they can be suitably represented in a tabular form. To this end, a template table may be provided, this table may also be used to display the same markings by the marking interface unit 10. When the user creates a marking, the marking may be added as an element of a table, based on for example the image dataset to which the marking belongs, or the date of that image dataset, and a characteristic of the marking. For example, when a number of markings relating to different predetermined body portions or locations in the image dataset are generated, these markings may be arranged according to location. For example, different dates in different rows and different locations in different columns. The role of columns and rows may be exchanged, of course.

The inserting unit 5 may be arranged for inserting the information at a position of the report 21 that depends on the marking. For example, different kinds of marking may be defined, wherein different kinds of marking are associated with different positions in the report. For example, the different positions correspond to different sections of the report. For example, different templates may be available for helping the user to create different kinds of markings to fulfill different kinds of information needs. These templates may also be associated with position information, to define the position where the information of the marking will be inserted should the user select the marking for insertion.

The system may comprise a note authoring unit arranged for enabling the user to author a note in respect of a marking selected for insertion. The inserting unit may then be arranged for, in response to the authoring of the note, inserting the note into the report and associating the note with said at least part of the marking in the report. Moreover, the layout of the inserted note in the report may be taken care of automatically.

The system may be implemented as a suitably programmed workstation. The workstation may be standalone, or the workstation may act as a terminal of a distributed computer system. Functionalities of the system as described herein may be implemented on different computing devices, in a distributed manner. It is also possible to implement all the described functionality in a single computing device.

The collection of markings may relate to a plurality of image datasets. Usually, a collection of markings relates to datasets in respect of the same subject, such as a patient. When the user opens an image dataset for creating markings and/or the report, the marking storing unit 2 may be arranged for retrieving a collection of existing markings of the same subject. These existing markings may relate to a different image dataset of the same subject. The collection of markings may be retrieved from a health record, for example. The marking storing unit 2 may further be arranged to add any newly created markings to the retrieved collection of existing markings. Moreover, the newly created markings may be stored together with the existing markers in the health record, for future reference. This way, the collection of markings may comprise historical information that can be used to quickly review disease progression.

Fig. 2 illustrates a method of creating a report.

In step 201, a collection of markings is stored. Herein, a marking 301 represents an annotation 302 and a set of viewing parameters 306 in respect of an image dataset. These markings may be obtained in any way, for example by enabling the user to create the markings in the way set forth herein.

In step 202, a user is enabled to create a report. While making the report, the user has inter alia two possibilities to use the markings. To this end, in step 203, the user is enabled to select a marking 301 of the collection of markings for display or insertion.

In step 204, it is determined whether the user has selected a marking 301 for display. In such a case, in step 205, the image dataset is displayed in dependence on the viewing parameters 306.

Otherwise, in step 206, it is determined whether the user has selected a marking for insertion. In such a case, in step 207, information is inserted into the report in dependence on the marking 301. For example, information from the annotation 302 of the marking 301 is extracted for insertion into the report.

After step 205 or step 207, the method continues from step 202, in which the user is enabled to continue creating the report further. However, if in step 202 it is determined that the report is complete, the report is stored and/or transmitted to an external device, and the method terminates.

The method may be implemented as a computer program product comprising instructions for causing a processor system to perform the method. Moreover, the method and computer program may be modified and/or extended by the skilled person, based on the functionality described herein in respect of the system.

It is possible that the system allows creation and handling of markings that are not associated with a particular image dataset, for example markings that relate to a palpation examination or a lab result. Such markings may comprise a reference to a data object or event to which the marking refers. Moreover, they may be used to insert information into the report based on the annotation 302 of the marking 301. Moreover, it is possible that some of the markers do not have viewing parameters associated therewith, although they do relate to an image dataset. In such a case, although any graphical object or indication of a measurement may still be displayed in a visualization of the image dataset, and the viewing parameters used when creating the marking may not be reproduced.

The collection of markings may comprise annotations in form of text, but also graphical annotations are possible. For example, if an annotation comprises a graphical object such as an arrow positioned at some position in the image dataset, the annotation could further comprise a schematic visualization of a structure shown in the image dataset (such as an organ), and the arrow. Such schematic visualization could be displayed by the marking interface unit 10 and/or inserted into the report 21 by the inserting unit 5.

The marking interface unit 10 could be arranged for displaying the collection of markings as a 'scratchpad' that comprises a document that may contain markings, and optional freeform descriptions. The marking interface unit 10 may further enable the user to manually arrange the order and presentation of the collection of markings in the 'scratchpad'.

The system and method may be regarded as a clinical decision support system and method. Such clinical decision support may assist radiologists in reporting medical images.

The following insights may be regarded relevant in radiology reporting:
- The moment of making annotations does not necessarily coincide with moment of creating the report in respect of a particular image dataset. For example, a radiologist may have been present during the acquisition of an image dataset and quickly made a measurement. At a later time, the same day or a later day, the radiologist may create the report to document the image dataset, including any findings and recommendations for the referring physician.
- Image markings/annotations may be created by another person as the person creating the actual report. For example, a resident may create the markings/annotations, while the radiologist may create the report.
- Some image markings may only serve as attention points for reporting, not all these markings/attention points need to be included in the report.

The system described herein may be configured to add a scratch area to the reporting display that contains the available attention points in the form of markings 301. These markings 301 may be manually created, generated by a computer-aided diagnosis system, or bookmarks. Additionally, each marking in the scratch area may trigger a (conference) presentation state to reproduce the visualization of the image in which the annotation was made. During the reporting process, these markings might be escalated to image findings by inserting information based on the annotation of a marking into the report. The link to the image position and presentation state may be preserved in the report. However, this is not a limitation. Some markings may only serve as attention points and may not be included in the report, for example because the radiologist finds them not important.

The solution described herein may be used to create a separation between a 'scratchpad' that contains a collection of markings, or preliminary attention points, and the report that contains official clinical information (e.g. approved by the radiologist).

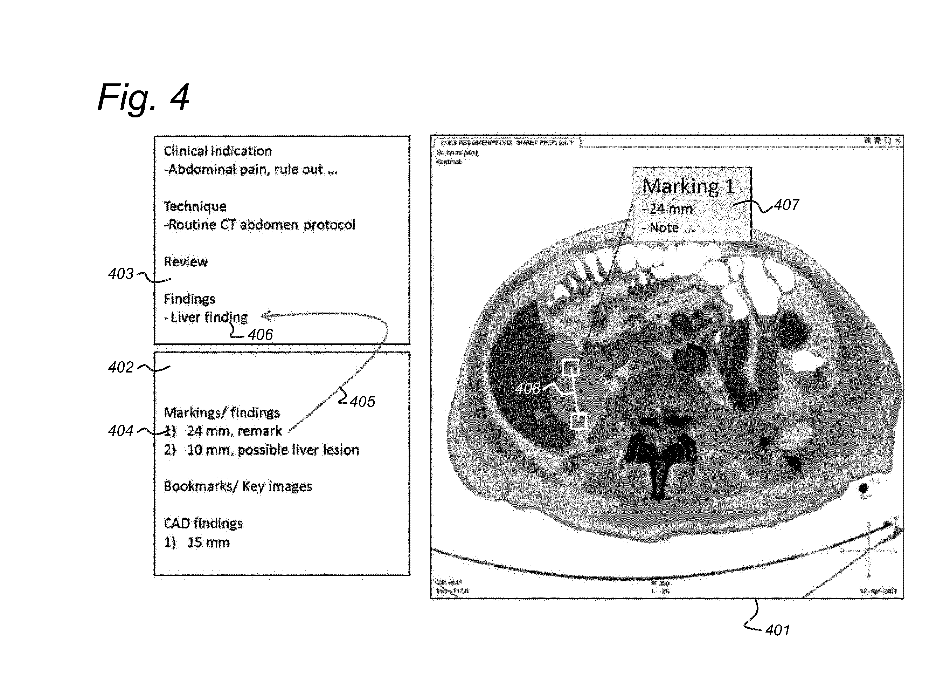

Fig. 4 shows a snapshot of a user interface screen generated by a system as described herein, during the phase of the creation of the report. The figure is only a sketch and may not be drawn to scale. The figure shows a display area 401 in which the image dataset is displayed. The figure further shows a display area 402 in which a collection of markings is displayed. This display area 402 may be labeled "Report scratch area" or may be given some other appropriate title. The user has selected the marking 404 for display. Consequently, the image display unit 4 has displayed a visualization of the image dataset, based on the viewing parameters 306 of the marking 404. Moreover, a graphical indication 408 of the measured object is drawn in the visualization of the image dataset. Also, some information 407 relating to the measurement is displayed.

Moreover, the user has selected the marking 404 for insertion. In the drawn embodiment, this has been done by means of a drag-and-drop operation schematically indicated by arrow 405.

The snapshot further comprises a report area 403 in which the report, insofar it has been created, is displayed. Information 406 has been inserted automatically into the report, in response to the user selecting the marking 404 for insertion. In the drawing, this information is "Liver finding". In practice, such information may be more detailed and include the measurement values.

In the shown example, the scratchpad has been organized according to whether the markings were created manually or by means of computer aided detection (CAD). Items 1) and 2) below "Markings/findings" were manual markings, whereas item 1) below "CAD findings" has been generated automatically.

While reporting, selected ones of the markings may be included in the report in one of the following ways. However, other scenarios are also possible and have been described elsewhere in this description.
- After selecting a marking for insertion, some remarks are dictated or typed by the user to report finding properties. These remarks may be also included into the report.
- In other cases, it may be appropriate to drag an image finding to the proper location in a table of lesions.

The scratch area may support the resident workflow in which the moment of making annotations does not coincide with moment of reporting. It may be used to separate the report preparation from the report creation task. Some image markings may only serve as attention points for reporting. The scratch area can be used as a communication tool to draw attention to an anatomical area for e.g. additional images. It may support the collaboration process and the personal workflow.

A report scratch area may be added to a radiology reporting display. There may be a direct link between the scratchpad and markings in the image; if the person reviewing the images (e.g., a radiologist or a resident) creates a marking, automatically a marking is created in the scratch area to which attention should be given. Thus, attention points such as user markings, CAD markings or bookmarks can be noted down. This aids the radiologist in preparing the report, which is usually done at a later stage.

This may support the workflow where a resident, rather than the radiologist himself, reviews the images and creates the markings. Moreover, the radiologist is provided with additional and/or more complete information available when dictating the report.

The scratchpad may contain an area for reporting findings (such as a table of findings) and items in the scratchpad can directly be escalated into findings. This further facilitates reporting. In tracking lesions / tumors, during reviewing images of the follow-up, the scratchpad may already contain findings / comments from the baseline (first scan) for easy reference and comparison. This further facilitates the follow-up reporting.

A system for creating a report may comprise a report unit for enabling a user to create a report; a marking storing unit for storing a collection of markings, wherein a marking represents an annotation and a set of viewing parameters in respect of an image dataset; a marking selection unit for enabling a user to select a marking of the collection of markings for display or insertion; an image display unit for, in response to the user selecting the marking for display, displaying the image dataset in dependence on the viewing parameters; and an inserting unit for, in response to the user selecting the marking for insertion, inserting information into the report in dependence on the marking.

It will be appreciated that the invention also applies to computer programs, particularly computer programs on or in a carrier, adapted to put the invention into practice. The program may be in the form of a source code, an object code, a code intermediate source and an object code such as in a partially compiled form, or in any other form suitable for use in the implementation of the method according to the invention. It will also be appreciated that such a program may have many different architectural designs. For example, a program code implementing the functionality of the method or system according to the invention may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise calls to each other. An embodiment relating to a computer program product comprises computer-executable instructions corresponding to each processing step of at least one of the methods set forth herein. These instructions may be sub-divided into sub-routines and/or stored in one or more files that may be linked statically or dynamically. Another embodiment relating to a computer program product comprises computer-executable instructions corresponding to each means of at least one of the systems and/or products set forth herein. These instructions may be sub-divided into sub-routines and/or stored in one or more files that may be linked statically or dynamically.

The carrier of a computer program may be any entity or device capable of carrying the program. For example, the carrier may include a storage medium, such as a ROM, for example, a CD ROM or a semiconductor ROM, or a magnetic recording medium, for example, a flash drive or a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. Use of the verb "comprise" and its conjugations does not exclude the presence of elements or steps other than those stated in a claim. The article "a" or "an" preceding an element does not exclude the presence of a plurality of such elements. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

## Claims

1. A system for displaying an image dataset, comprising
a marking storing unit (2) for storing a collection of markings, wherein a marking (301) represents an annotation (302) and a set of viewing parameters (306) in respect of an image dataset;
a marking selection unit (3) for selecting a marking (301) of the collection of markings for display; and
an image display unit (4) for, in response to the marking (301) being selected for display, displaying the image dataset in dependence on the viewing parameters (306) of the marking (301) selected for display.

2. The system according to claim 1, wherein the marking selection unit (3) is arranged for selecting the marking (301) of the collection of markings for insertion; and
the system further comprising
a report unit (1) for creating a report (21); and
an inserting unit (5) for, in response to the marking (301) being selected for insertion, inserting information into the report in dependence on the marking (301).

3. The system according to claim 1, comprising
a marking creation unit (6) for creating a marking (301) while the image dataset is displayed, and providing the marking (301) to the marking storage unit (2) for storage,
wherein the marking creation unit (6) comprises an annotation creation unit (7) for enabling the user to indicate the annotation (302) and a parameter unit (8) for determining the set of viewing parameters (306) of the marking based on a displayed view of the image dataset.

4. The system according to claim 3, wherein the marking creation unit (6) comprises a template (9) for enabling the user to provide information in respect of a marking in a structured form.

5. The system according to claim 3, wherein the system supports a first operating mode in which the marking creation unit (6) is enabled and the inserting unit (5) is disabled, and a second operating mode in which the inserting unit (5) is enabled.

6. The system according to claim 1, comprising a marking interface unit (10) for displaying at least part of the collection of markings, and wherein the marking selection unit (3) is arranged for enabling the user to perform the selection of a marking via the marking interface unit (10).

7. The system according to claim 6, wherein the marking interface unit (10) is arranged for displaying markings in respect of a plurality of different image datasets, and for displaying an indication of which marking belongs to which image dataset.

8. The system according to claim 1, wherein the annotation (302) comprises a graphical object (303) or a measurement (304) defined in respect of the image dataset, and wherein the display unit (4) is arranged for, in response to the marking (301) being selected for display, displaying the graphical object (303) or measurement (304) in association with the image dataset.

9. The system according to claim 2, wherein the annotation (302) comprises a text fragment (305), and the inserting unit (5) is arranged for pasting the text fragment (305) into the report (21) in response to the marking (301) being selected for insertion.

10. The system according to claim 2, wherein the annotation (302) comprises a measurement (304) defined in respect of the image dataset, and wherein the inserting unit (5) is arranged for pasting information relating to the measurement (304) into the report (21) in response to the marking (301) being selected for insertion.

11. The system according to claim 2, wherein the inserting unit (5) is arranged for inserting the information into the report as a structured data element or for inserting information based on a plurality of markings into the report in form of at least part of a table.

12. The system according to claim 2, wherein the inserting unit (5) is arranged for inserting the information at a position of the report that depends on the marking (301).

13. A workstation comprising the system according to claim 1.

14. A method of displaying an image dataset, comprising
storing (201) a collection of markings, wherein a marking (301) represents an annotation (302) and a set of viewing parameters (306) in respect of an image dataset;
selecting (203) a marking (301) of the collection of markings for display or insertion;
in response to the marking (301) being selected for display (204), displaying (205) the image dataset in dependence on the viewing parameters (306) of the marking (301) selected for display.

15. A computer program product comprising instructions for causing a processor system to perform the method according to claim 13.
